**Europäisches Patentamt**

**European Patent Office**

**Office européen des brevets**

(19)

(11) Veröffentlichungsnummer : **0 005 263**
**B1**

(12) # EUROPÄISCHE PATENTSCHRIFT

(45) Veröffentlichungstag der Patentschrift :
**11.02.81**

(21) Anmeldenummer : **79101330.3**

(22) Anmeldetag : **02.05.79**

(51) Int. Cl.³ : **C 07 C 21/10**, C 07 C 17/24,
B 01 J 23/72

(54) Verfahren zur Herstellung von Trichloräthylen.

(30) Priorität : **02.05.78 DE 2819209**

(43) Veröffentlichungstag der Anmeldung :
**14.11.79 (Patentblatt 79/23)**

(45) Bekanntmachung des Hinweises auf die Patenterteilung : **11.02.81 Patentblatt 81/06**

(84) Benannte Vertragsstaaten :
**BE CH DE FR GB IT NL SE**

(56) Entgegenhaltungen :
DE - B - 1 194 403
US - A - 2 967 835

(73) Patentinhaber : **WACKER-CHEMIE GMBH**
**Prinzregentenstrasse 22**
**D-8000 München 22 (DE)**

(72) Erfinder : **Mack, Wilhelm, Dr.Dipl.-Chem**
**Unghausen 24**
**D-8263 Burghausen (DE)**
Erfinder : **Fruhwirth, Otto, Dr.Dipl.-Chem**
**Ludwig-Thoma-Strasse 25**
**D-8263 Burghausen (DE)**
Erfinder : **Strasser, Rudolf, Dr. Dipl.-Chem**
**Lindacher Strasse 58**
**D-8263 Burghausen (DE)**
Erfinder : **Schmidhammer, Ludwig, Dr.Dipl.-Chem**
**Pappelweg 5**
**·D-8261 Halming/Marktl (DE)**
Erfinder : **Pichl, Eduard**

**D-8261 Mehring 7 (DE)**

Imprimerie Jouve, 17, rue du Louvre, 75001 Paris, France

EP 0 005 263 B1

# 0 005 263

## Verfahren zur Herstellung von Trichloräthylen

Gegenstand der Erfindung ist ein Verfahren zur Herstellung von Trichloräthylen aus Tetrachloräthylen und Wasserstoff vermittels eines eine Kupferverbindung enthaltenden Trägerkatalysators bei erhöhter Temperatur.

Es ist bereits bekannt, Tetrachloräthylen mit Wasserstoff in Gegenwart von auf Aluminiumoxid verteilten Kupfersalzen zu Trichloräthylen umzusetzen. Auch der Zusatz von Alkalimetallsalzen, wie Kalium-, Rubidium- und Caesiumsalzen ist bereits beschrieben. In der DE-AS 11 94 403 wird jedoch darauf hingewiesen, daß das System Aluminiumoxid/Kupfersalz nur geringe und schlecht reproduzierbare Umsetzungen ergibt. Auch die Aktivierung mit Alkalisalzen erbringt auch bei Temperaturen von 200 bis 325 °C nur eine bescheidene Verbesserung der Umsetzung.

Aufgabe der Erfindung war es, ein Verfahren zu finden, das bei niedriger Reaktionstemperatur gute Ausbeuten und hohe Selektivität ermöglicht.

Gelöst wird diese Aufgabe durch ein Verfahren, welches dadurch gekennzeichnet ist, daß Tetrachloräthylen in einer Menge von 0,5 bis 5 Mol pro Stunde und pro Liter Kontaktmasse über einen Katalysator, bestehend aus Aktivkohleträger mit mehr als 500 m$^2$/g BET-Oberfläche, 0,5 bis 20 Gew.% Kupfer in elementarer oder chemisch gebundener Form, und 0,01 bis 1 Gew.% eines oder mehrerer der Metalle Palladium, Ruthenium oder Rhodium in elementarer oder chemisch gebundener Form bei Temperaturen zwischen 150 und 250 °C und einem Wasserstoffdruck von 1 bis 100 bar zur Reaktion gebracht wird.

Die neu entwickelten Katalysatoren zeigen überraschenderweise gegenüber herkömmlichen Katalysatoren hervorragende Eigenschaften. Es war keineswegs zu erwarten, daß bei Änderung der Katalysatorträgermasse von Aluminiumoxid zu Aktivkohle mit Oberflächen über 500 m$^2$/g und Zusatz von 0,01 bis 1 Gew.% eines oder mehrerer der Metalle Palladium, Ruthenium oder Rhodium in elementarer oder chemisch gebundener Form die Selektivität, der Umsatz und die Raum-Zeit-Ausbeute so drastisch verbessert werden konnten. Der neu entwickelte Katalysator besteht aus Aktivkohle als Trägermaterial mit einer BET-Oberfläche über 500 m$^2$/g, solche mit Oberflächen bis 1400 m$^2$/g sind handelsüblich. Die Aktivkohle wird üblicherweise in gekörnter Form in Korngrößen zwischen 2 und 10 mm Durchmesser eingesetzt. Der Katalysator ist mit feinverteilten Salzen von einem oder mehreren der Metalle Palladium, Ruthenium oder Rhodium sowie Kupfer belegt. Als Salze der genannten Metalle kommen alle gut wasser- und säurelöslichen anorganischen bzw. organischen in Frage, wobei die für die Bereitung des erforderlichen Katalysators von z.B. 1 kg notwendigen Salzmengen in 0,5 bis 10 l Wasser löslich sein sollten. Der Katalysatorträger ist mit 0,5 bis 20 Gew.% Kupfer in elementarer oder chemisch gebundener Form, und 0,01 bis 1 Gew.% eines oder mehrerer der Metalle Palladium, Ruthenium oder Rhodium in elementarer oder chemisch gebundener Form auf der Oberfläche belegt. Zu Beginn bzw. während der Reaktion tritt eine teilweise bzw. vollständige Reduktion der Kupfer- bzw. Palladium-, Ruthenium- oder Rhodiumsalze zu deren Metallen ein.

Die Herstellung des Katalysators erfolgt beispielsweise in der Weise, daß 160 g gekörnte Aktivkohle (BET-Oberfläche > 500 m$^2$/g) eines handelsüblichen Produktes mit 140 ml einer waßrigen Lösung getränkt bzw. besprüht und anschließend bei 160 °C im Stickstoffstrom getrocknet wird, die 260 mg Palladiumchlorid und 43 g Kupferchloriddihydrat gelöst enthält. Die Behandlung der Aktivkohle kann ebenfalls jeweils mit den getrennten Salzlösungen nacheinander erfolgen.

Die Bereitung des neu entwickelten Katalysators ist gegenüber dem Stand der Technik wesentlich vereinfacht, wie er in der DE-OS 11 94 403 gegeben ist. Die dort erforderliche, umständliche und gefährliche Behandlung mit Wasserstoff und Sauerstoff bei 300 °C entfällt beim erfindungsgemäßen Kontakt gänzlich.

Vorzugsweise werden Katalysatoren mit Kupfergehalten zwischen 3 und 15 Gew.% eingesetzt. Der fertige Katalysator hat ein Schüttgewicht zwischen 200 und 600 g/Liter.

Die Aktivität des Katalysators hängt fast linear vom Gehalt eines oder mehrerer der Metalle Palladium, Ruthenium oder Rhodium ab. So ändert sich beispielsweise unter vergleichbaren Bedingungen beim Übergang von 0,01 % Palladiumgehalt auf 0,1 % Palladiumgehalt im neu entwickelten Katalysator die Ausbeute am gewünschten Produkt um das 10-fache, bei einem Gehalt von 0,2 % Palladium wird nochmals die Ausbeute fast verdoppelt. Bei weiterer Steigerung des Palladiumgehaltes steigt der Umsatz und die Ausbeute weiter an, wobei schon ab 0,8 % Palladium ein Ausbeuteplateau erreicht ist.

Zur Durchführung des Verfahrens wird der neu entwickelte Katalysator in geschütteter Form in ein Reaktionsrohr gebracht. Zur Reaktion wird das bei der Reaktionstemperatur gasförmige Tetrachloräthylen in Mengen von 0,5 bis 5 Mol pro Stunde und pro Liter Kontaktmasse, zusammen mit der 0,1- bis 1-fachen äquimolaren Menge Wasserstoffgas zur Reaktion gebracht. Die Reaktion verläuft schon bei Temperaturen über 150 °C merklich, bei 180 °C werden in der Anfangsphase der Katalysatorstandzeit bereits Umsätze von 98 bis 99 % der Theorie erreicht, während mit Katalysatoren gemäß dem Stand der Technik nur bis maximal 95 % erreicht werden können. Die Raum-Zeit-Ausbeuten liegen bereits bei durchschnittlich 50° tieferen Temperaturen gegenüber dem aus dem Stand der Technik bekannten Verfahren bei wesentlich höheren Werten. Reaktionstemperaturen über 250 °C sind im allgemeinen nicht erforderlich. Ein weiterer Vorteil des Verfahrens liegt darin, daß im Gegensatz zum bekannten Verfahren auch bei erhöhtem Wasserstoffdruck gearbeitet werden kann. Zweckmäßigerweise wird bei Drücken zwischen 1 und 100 bar gearbeitet, wobei Drücke zwischen 1 und 10 bar, insbesondere 3 bis 10 bar, häufig vorteilhaft sind. Der bei der Verfahrensweise unter Druck anfallende Chlorwasserstoff kann ohne vorherige Verdichtung in anderen geeigneten Verfahren, wie z.B. der Oxychlorierung, eingesetzt werden. Die Abscheidung der Reaktionsprodukte wird ebenfalls wesentlich vereinfacht.

Wenn der Katalysator über längere Zeit eingesetzt wird, kann ein Aktivitätsabfall durch Erhöhung der Temperatur ausgeglichen werden. Zweckmäßig beginnt man die Reaktion bei niedriger Temperatur, wie z.B. 180 °C, und steigert erst langsam die Temperatur bei beginnendem Aktivitätsverlust. Die Haltbarkeit der neu entwickelten Katalysatoren liegt bei über 1 Jahr.

## Beispiel 1

160 g gekörnte Aktivkohle (Körnung 2 bis 10 mm) der Bezeichnung EKT-IV, Fa. Lurgi, mit einer Oberfläche von 1200 $m^2$/g wurde mit 140 ml einer wäßrigen Lösung von 260 mg (0,1 Gew. % Pd) Palladiumchlorid und 43 g (10 Gew. % Cu) Kupferchloriddihydrat getränkt. Nach einstündigem Trocknen bei 160 °C im Stickstoffstrom ist der Katalysator gebrauchsfertig.

## Beispiel 2 (Vergleichsversuch, DE-AS 11 94 403)

335 g Aluminiumoxid mit BET-Oberfläche 190 $m^2$/g wurden mit einer Lösung aus 32 g Kupferchloriddihydrat und 150 g Kaliumchlorid in 450 ml Wasser getränkt, getrocknet und bei 290 °C zuerst 30 Minuten mit Sauerstoff und anschließend nach einer Zwischenspülung mit Stickstoff 30 Minuten mit Wasserstoff nachbehandelt.

## Beispiel 3 (Vergleichsversuch)

Auf einem Aktivkohleträger gemäß Beispiel 1 wurde 0,1 Gew. % Palladium in Form von Palladiumchlorid aufgebracht.

## Beispiel 4 (Vergleichsversuch)

Auf einem Aktivkohleträger gemäß Beispiel 1 wurde 10 Gew. % Kupfer als Kupferchlorid aufgebracht. Unter gleichen Reaktionsbedingungen wie in Beispiel 1 wurde nur geringer Umsatz zu Trichloräthylen erzielt.

## Beispiel 5

151 g gekörnte Aktivkohle entsprechend Beispiel 1 wurde mit einer Lösung von 42,2 g (10 Gew. % Cu) Kupferchlorid ($CuCl_2.2H_2O$) und 385 mg (0,044 Gew. % Rh) Natriumhexachlororhodinat ($Na_3RhCl_6.12H_2O$) in 130 ml Wasser, getränkt. Nach einstündigem Trocknen bei 160 °C war der Katalysator gebrauchsfertig.

## Beispiel 6

151 g gekörnte Aktivkohle entsprechend Beispiel 1 wurde mit einer Lösung von 42,2 g (10 Gew. % Cu) Kupferchlorid ($CuCl_2.2H_2O$) und 366 mg (0,1 Gew. % Rh) Rutheniumtrichlorid ($RuCl_3.2H_2O$) in 130 ml Wasser getränkt. Nach einstündigem Trocknen bei 160 °C ist der Katalysator gebrauchsfertig.

## Beispiel 7

151 g gekörnte Aktivkohle entsprechend Beispiel 1 wurde mit einer Lösung von 42,2 g (10 Gew. % Cu) Kupferchlorid ($CuCl_2.2H_2O$) und 175 mg (0,02 Gew. % Rh) Natriumhexychlororhodinat ($Na_3RhCl_6.12H_2O$) in 130 ml Wasser getränkt. Nach einstündigem Trocknen bei 160 °C war der Katalysator gebrauchsfertig.

## Beispiel 8

151 g gekörnte Aktivkohle entsprechend Beispiel 1 wurde mit einer Lösung von 21,1 g (5 Gew. % Cu) Kupferchlorid ($CuCl_2.2H_2O$) und 260 mg (0,1 Gew. % Pd) Palladiumchlorid in 130 ml Wasser getränkt.

Herstellung von Trichloräthylen unter Einsatz der in den Beispielen angeführten Katalysatoren
(Wenn nicht gesondert vermerkt, wurde bei einem Wasserstoffdruck von 1 bar gearbeitet)

| Katalysator | | Perchloräthylen-zufuhr in Gramm pro Liter Kata-lysator pro Stunde | Wasserstoffzu-fuhr in Litern pro Liter Kon-takt pro Stunde | Reaktions-Temperatur °C | Umsetzung zu % Trichloräthylen im Reaktions-produkt | Raum-Zeit-Ausbeute in g Trichloräthylen pro Liter Kontakt pro Stunde |
|---|---|---|---|---|---|---|
| gemäß Bei-spiel 2 | a | 130 | 22,4 | 180 | 0,7 | 0,9 |
| | b | 130 | 22,4 | 250 | 3,7 | 5,1 |
| | c | 19 | 6,6 | 250 | 17,7 | 3,3 |
| | d | 8,9 | 2,4 | 250 | 33,2 | 2,4 |
| gemäß Bei-spiel 1 | a 1) | 130 | 22,4 | 180 | 31,8 | 41,5 |
| | b 2) | 324 | 20 | 193 | 41 | 120 |
| gemäß Bei-spiel 3 | 3) | 166 | 22,8 | 180 | 4,0 | 5,1 |
| gemäß Bei-spiel 4 | | 130 | 22,8 | 180 | 1,9 | 2,6 |
| gemäß Bei-spiel 5 | a 4) | 130 | 22,8 | 180 | 31 | 37 |
| | b | 130 | 22,8 | 200 | 61 | 70 |
| | c 5) | 324 | 20 | 220 | 40 | 115 |
| gemäß Bei-spiel 6 | a | 130 | 22,4 | 180 | 6,5 | 7,8 |
| | b | 130 | 22,4 | 200 | 15,4 | 18,5 |
| gemäß Bei-spiel 7 | | 130 | 22,8 | 180 | 21 | 26 |
| gemäß Bei-spiel 8 | | 130 | 22,8 | 180 | 27 | 33 |

Nach einstündigem Trocknen bei 160 °C ist der Katalysator gebrauchsfertig.

Aus den Beispielen geht klar die Überlegenheit des erfindungsgemäßen Verfahrens unter Einsatz der neu entwickelten Katalysatoren hervor.

1) Gemäß Beispiel 1, a, entstanden lediglich 0,3 g cis-, 0,06 g trans-Dichloräthylen, 0,08 g Vinylidenchlorid, 0,023 g Äthylen und 0,002 g Äthan.

2) Hier wird bei einem Wasserstoffdruck bei 5 bar gearbeitet. Es entstehen 1,18 g cis-, 0,38 g trans-Dichloräthylen sowie 0,36 g Vinylidenchlorid.

3) Als Nebenprodukte entstehend 0,3 g cis-Dichloräthylen, 0,15 g Vinylidenchlorid, 0,79 g Äthan und 0,74 g Äthylen.

4) Als Nebenprodukt entstehen 0,015 g Vinylidenchlorid, 0,17 g cis-Dichloräthylen, 0,023 g trans-Dichloräthylen, 1,4 mg Äthylen und 0,1 mg Äthan.

5) Hier wird bei einem Wasserstoffdruck von 10,5 bar gearbeitet. Als Nebenprodukte entstehen 0,17 g Vinylidenchlorid, 1,2 g cis-Dichloräthylen und 0,24 g trans-Dichloräthylen.

**Anspruch**

Verfahren zur Herstellung von Trichloräthylen aus Tetrachloräthylen und Wasserstoff vermittels eines eine Kupferverbindung enthaltenden Trägerkatalysators bei erhöhter Temperatur, dadurch gekennzeichnet, daß Tetrachloräthylen in einer Menge von 0,5 bis 5 Mol pro Stunde und pro Liter Kontaktmasse über einen Katalysator, bestehend aus Aktivkohleträger mit mehr als 500 m²/g BET-Oberfläche, 0,5 bis 20 Gew. % Kupfer in elementarer oder chemisch gebundener Form und 0,01 bis 1 Gew. % eines oder mehrerer der Metalle Palladium, Ruthenium oder Rhodium in elementarer oder chemisch gebundener Form bei Temperaturen zwischen 150 und 250 °C und einem Wasserstoffdruck zwischen 1 bis 100 bar zur Reaktion gebracht wird.

**Claim**

Process for the manufacture of trichloroethylene from tetrachloroethylene and hydrogen, at elevated temperature, by means of a carrier catalyst containing a copper compound, characterised in that tetrachloroethylene is brought to reaction in a quantity of from 0.5 to 5 moles per hour and per litre of contact composition over a catalyst comprising an activated carbon carrier having a BET specific surface area of more than 500 m²/g, 0.5 to 20 % by weight of copper in elemental or chemically bonded form, and 0.01 to 1 % by weight of one or more of the metals palladium, ruthenium and rhodium in elemental or chemically bonded form, at a temperature of from 150 to 250 °C and a hydrogen pressure of from 1 to 100 bar.

**Revendication**

Procédé de préparation du trichloréthylène à partir du tétrachloréthylène et de l'hydrogène au moyen d'un catalyseur contenant un composé du cuivre, à température élevée, procédé caractérisé en ce qu'on fait réagir le tétrachloréthylène en une quantité de 0,5 à 5 moles par heure et par litre de masse catalytique, sur un catalyseur constitué d'un support en charbon actif ayant une surface BET supérieure à 500 m²/g, de 0,5 à 20 % en poids de cuivre sous forme élémentaire ou chimiquement liée et de 0,01 à 1 % en poids d'un ou plusieurs des métaux palladium, ruthénium et rhodium sous forme élémentaire ou chimiquement liée, à des températures comprises entre 150 et 250 °C et sous une pression d'hydrogène comprise entre 0,1 et 10 MPa.